# EUROPEAN PATENT SPECIFICATION

(11) **EP 3 478 347 B1**
(45) Date of publication and mention of the grant of the patent: **01.12.2021**
(21) Application number: 17733477.8
(22) Date of filing: 29.06.2017
(51) Int. Cl.: A61M 11/00, A61F 9/00, B05B 17/06

(54) **AN AEROSOL GENERATOR**
AEROSOLERZEUGER
GÉNÉRATEUR D'AÉROSOL

(30) Priority: 04.07.2016 EP 16177850
(43) Date of publication of application: 08.05.2019
(73) Proprietor: Stamford Devices Limited, Dangan, Galway (IE)
(72) Inventor: KELLY, Patrick Martin, County Clare (IE); GREHAN, Joseph, County Galway (IE); MAGUIRE, Finbarr, County Clare (IE)
(74) Representative: Weldon O'Brien Ltd.
(86) International application number: PCT/EP2017/066225
(87) International publication number: WO 2018/007245

(56) References cited:
- WO-A1-2012/009706
- WO-A1-2013/090468
- JP-A- 2009 072 313
- US-A1- 2009 192 443

## Description

### Introduction

This invention relates to an aperture plate for generating an aerosol and to an aerosol generator incorporating the aperture plate according to the claims.

The aerosol generator is particularly suitable for delivery of an aerosol of ophthalmic fluid to the eye.

Aerosol delivery of ophthalmic fluid to the eye is described in US8012136 and US8684980. There are a number of problems with known systems of this type. It is difficult to deliver a sufficient amount of the aerosol with effective droplet size sufficiently quickly before involuntary blink reflex and/or lacrimation reflex occurs.

Aerosol generators comprising a vibratable member and a plate body operably coupled to the vibratable member are known. The plate body has a top surface, a bottom surface, and a plurality of apertures extending from the top surface to the bottom surface. The apertures may be tapered such that when a liquid is supplied to one surface and the aperture plate is vibrated using the vibratable member, liquid droplets are ejected from the opposite surface. Details of such known systems are described for example in US6235177, US20070023547A, and US7066398 JP2009-072313 describes an ophthalmic drug spraying and dispensing apparatus.

WO2012/009706A describes a device for generating droplets and delivering to the eye.

### Statements of Invention

According to the invention there is provided an aerosol generator as defined in claim 1.

In one embodiment the aperture plate is substantially domed shaped in geometry.

In one case the apertures are tapered to narrow from the inlet opening at the inlet surface to an outlet opening adjacent to the outlet surface. There may be a flared portion adjacent to the exit opening.

The invention also provides an aerosol generator comprising an aperture plate of the invention.

In one embodiment the aerosol generator comprises:-
a housing having an inlet for receiving a liquid to be aerosolised and an outlet through which the aerosol is delivered;
a support member for the aperture plate; and
a vibration generating element for vibrating the aperture plate.

The aerosol generator may comprise a reservoir for liquid to be aerosolised.

In one case the reservoir is defined by a portion of the housing.

In one case the duration of the on-off cycle is less than 20 microseconds.

In one embodiment the support member comprises a central opening and the aperture plate is mounted to the underside of the support member.

The invention also provides an ophthalmic fluid delivery device comprising an aerosol generator of the invention.

The aperture plate may be of any suitable thickness. In one case the aperture plate has a thickness of from 59 to 63 microns.

The invention also provides an aerosol generator comprising an aperture plate of the invention and means for vibrating the aperture plate. The means for vibrating the aperture plate is preferably configured to vibrate the plate at a frequency of from 125 to 155 kHz. The plate may be vibrated at from 128 to 148 kHz.

In one case the aperture plate comprises a plate body having a top surface, a bottom surface, and a plurality of apertures that taper in a direction from the top surface to the bottom surface. Liquid is supplied to the top (rear) surface of the aperture plate, and the aperture plate is vibrated to eject liquid droplets from the bottom (front) surface. Further, the apertures have an exit angle that is in the range from about 30° to about 60°, more preferably about 41° to about 49°, and more preferably at about 45°. The apertures may have a diameter that is in the range from about 1 micron to about 10 microns at the narrowest portion of the taper. Such an aperture plate is advantageous in that it may produce liquid droplets having a size that are in the range from about 2 to 10 microns.

In some cases, in order to produce larger droplets, the outlet openings are at least 10µm in diameter. Such droplets avoid deflection by the eye.

In some cases the outlet openings are from 10µm to 20µm, or from 15µm to 20µm in diameter. In one embodiment the outlet openings are about 20µm in diameter.

Techniques for vibrating such aperture plates are described generally in U.S. Pat. Nos. 5,164,740; 5,586,550; and 5758637. The aperture plates are constructed to permit the production of relatively small liquid droplets at a relatively fast rate.

To enhance the rate of droplet production while maintaining the droplets within a specified size range, the apertures may be constructed to have a certain shape. More specifically, the apertures are preferably tapered such that the aperture is narrower in cross section where the droplet exits the aperture. In one case, the angle of the aperture at the exit opening (or the exit angle) is in the range from about 30° to about 60°, more preferably from about 41° to about 49°, and more preferably at about 45°. Such an exit angle provides for an increased flow rate while minimizing droplet size. In this way, the aperture plate may find particular use with inhalation drug delivery applications.

In some cases may have an exit opening having a diameter in the range from about 1 micron to about 10 microns, to produce droplets that are about 2 microns to about 10 microns in size. The taper at the exit angle is preferably within the desired angle range for at least about the first 15 microns of the aperture plate. Beyond this point, the shape of the aperture is less critical. For example, the angle of taper may increase toward the opposite surface of the aperture plate.

The aperture plate is typically constructed from a palladium nickel alloy. Such an alloy is corrosion resistant to many corrosive materials particularly solutions for treating respiratory diseases by inhalation therapy, such as an albuterol sulfate and ipratropium solution, which is used in many medical applications. Further, the palladium nickel alloy has a low modulus of elasticity and therefore a lower stress for a given oscillation amplitude.

The aperture plates of the invention may be constructed using an electro-deposition process where a metal is deposited from a solution onto a conductive mandrel by an electrolytic process. In one example, the aperture plates are formed using an electroforming process where the metal is electroplated onto an accurately made mandrel that has the inverse contour, dimensions, and surface finish desired on the finished aperture plate. When the desired thickness of deposited metal has been attained, the aperture plate is separated from the mandrel. Electroforming techniques are described generally in E. Paul DeGarmo, "Materials and Processes in Manufacturing" McMillan Publishing Co., Inc., New York, 5.sup.th Edition, 1979.

### Brief Description of the Drawings

The invention will be more clearly understood from the following description thereof, given by way of example only, in which:-
Fig. 1 is a cross sectional view of a prior art aperture plate;
Fig. 2 is an enlarged view of one of the apertures of the plate of Fig. 1;
Fig. 3 is a cross sectional view of an aperture plate according to the invention;
Fig. 4 is an enlarged view of one of the apertures of the plate of Fig. 3;
Fig. 5 is a cross sectional view of a prior art aerosol generator;
Fig. 6 is an enlarged view of portion of the prior art aerosol generator of Fig. 5 showing a diverging aerosol plume;
Fig. 7 is a cross sectional view of an aerosol generator according to the invention;
Fig. 8 is an enlarged view of the aerosol generator of Fig. 7 showing a converging plume;

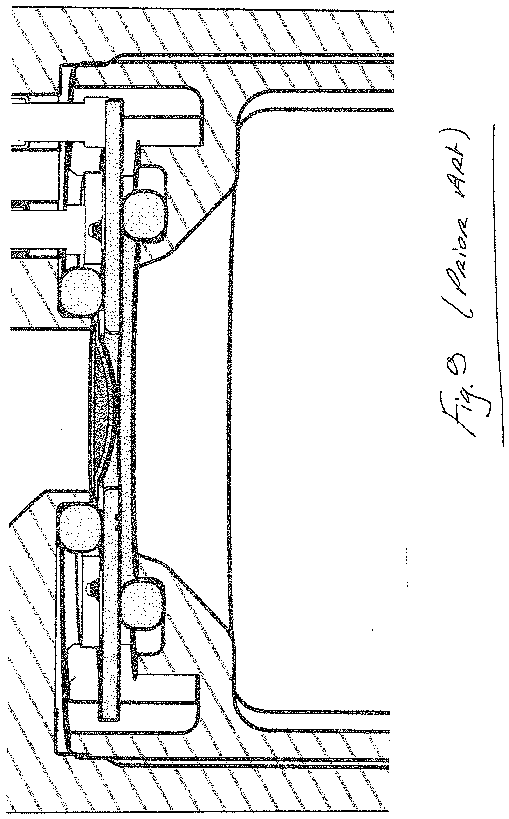

Fig. 9 is an enlarged view of portion of Fig. 5 showing the mounting of a prior art aperture plate;

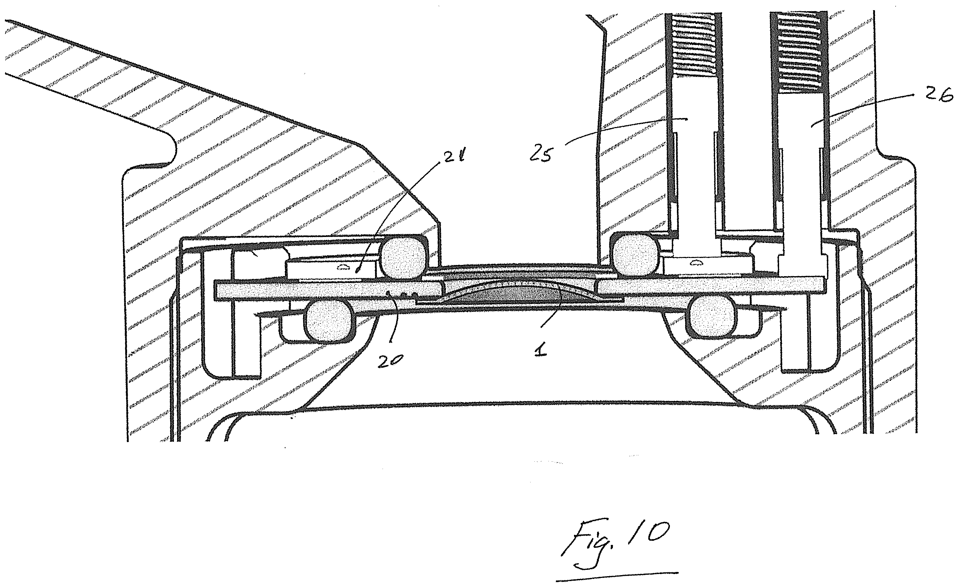

Fig. 10 is an enlarged view of portion of Fig. 7 showing the mounting of an aperture plate of the invention;
Fig. 11 is an image of a plume generated using a prior art aerosol generator;
Fig. 12 is an image of a plume generator using an aerosol generator of the invention; and
Fig. 13 is an image of pulsed aerosol flow showing individual droplets.

### Detailed Description

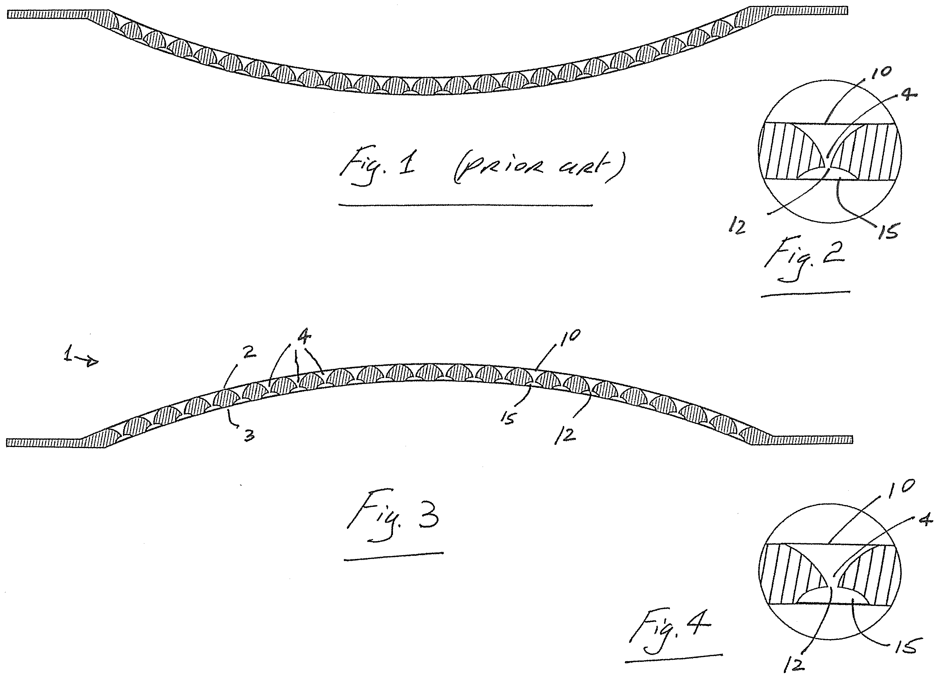

Referring initially to Figs. 3 and 4 there is illustrated an aperture plate 1 according to the invention which comprises an inlet surface 2 for receiving a liquid to be aerosolised, an outlet surface 3 and a plurality of apertures 4 extending therebetween. The apertures 4 are tapered from an inlet opening 10 at the inlet surface 2 to an outlet or exit opening 12. In this case there is a flared portion 15 leading from the exit opening. It will be noted that the aperture plate is dome shaped in geometry. In the invention the aperture plate 1 is convex in the direction of the inlet openings 10 and concave in the direction of the exit openings 12. The aerosol exits on the concave side of the dome-shaped aperture plate.

There is in contrast to prior art aperture plates as illustrated in Fig. 1 which are domed in the opposite or inverse direction, convex in the direction of the outlet openings 12 and concave in the direction of the inlet openings 10. In the prior art the aerosol exits on the convex side of the plume- shaped aperture plate.

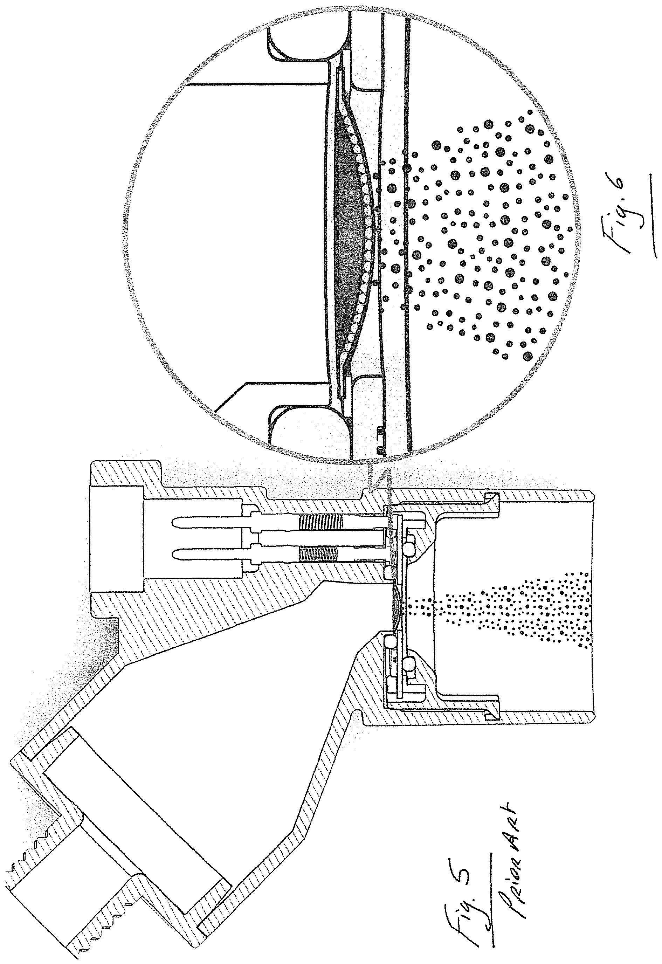

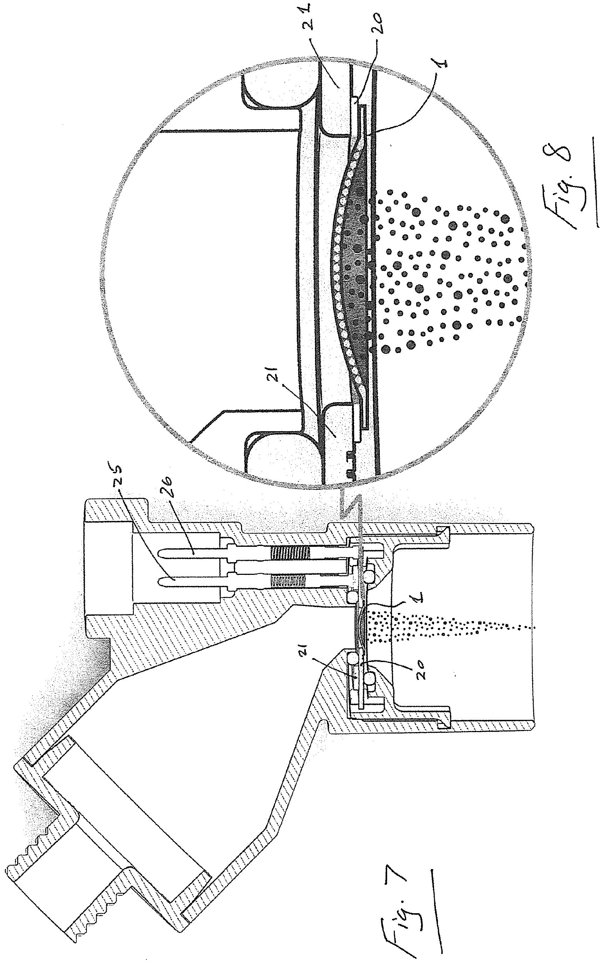

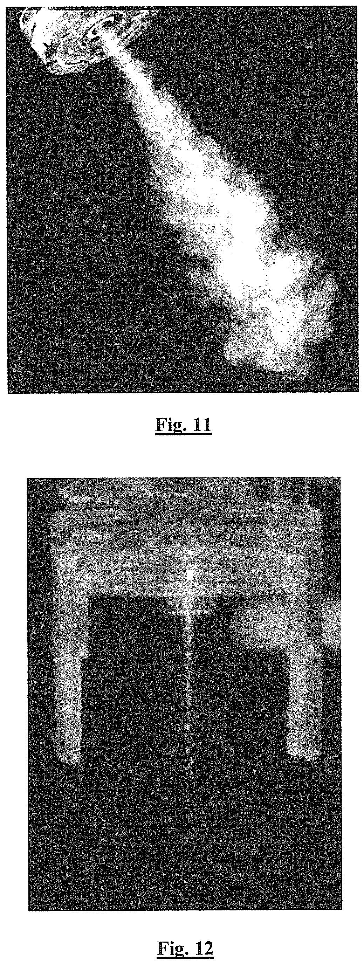

We have found that this difference is highly significant. In the case of the prior art aperture plate a diverging aerosol plume is generated as illustrated in Figs. 5, 6 and 11. In contrast, the aperture plate of the invention generates an aerosol plume which converges as illustrated in Figs. 7, 8 and 12.

The aperture plate of the invention may be incorporated in any suitable aerosol generator system such as that described in our WO2012/04622A (Aerogen Solo II). The arrangement illustrated in the drawings is given by way of example only.

The aperture plate 1 is mounted to an annular support member or washer 20 which has a central opening. A vibration generating element, in this case an annular shaped piezo 21, is used to vibrate the aperture plate 1. A first electrical power conducting pin 25 engages with a first surface of the piezo 21 and a second conducting pin 26 conducts electrical power to a second surface of the piezo 21.

In the case of the aperture plate of the invention the aperture plate 1 is mounted to the underside of the support washer 20. This assists in creating a reservoir area above the convex (inlet) side of the aperture plate which enhances the efficiency of aerosolisation.

### Example 1

An aerosol generator of the type described in our WO2012/046220A (Aerogen Solo II) was used in this experiment. Two different types of aperture plates were used:-
A - a prior art aperture plate which is domed such that the aerosol exits on the convex side of the plate;
B - an aperture plate of the invention which is domed so that the aerosol exits on the concave side of the plate.

Type A aperture plates with 10µm outlet openings were evaluated and found to have % dose recovery of 60-75% at a distance of 3 cm and through a 6 mm aperture.

Two Type B 10 µm inversely domed aperture plates were also evaluated through the same setup. The inverse domed Type B aperture plates showed a large increase in dose recovery. The 10 µm INV2-S1 and 10 µm INV2-S2 had recovered doses of 88-99.8 % and 90-99.6 % respectively.

Type A aperture plates with 15µm outlet openings were also evaluated and were found to have % dose recovery of 65-83% at a distance of 3 cm and through a 6 mm aperture.

Type B 15 µm inversely domed aperture plates were also evaluated through the same setup. The inversely domed aperture plates showed a large increase in dose recovery. The 15 µm INV2-S2 had recovered doses of 92-98.4%.

Type A aperture plates with 20µm outlet openings were evaluated and found to have % dose recovery of 65-73% at a distance of 3 cm and through a 6 mm aperture.

Two Type B 20 µm inversely domed aperture plates were also evaluated through the same setup. The inverse domed Type B aperture plates showed a large increase in dose recovery. The 20 µm INV2-S1 and 20 µm INV2-S2 had recovered doses of 92-99.1% and 92-99% respectively.

From this experiment it is clear that the doming profile has a large influence on dose delivery. It was observed that aperture plates Type A with the regular dome profile, the average dose recovery was -60-75% while the aperture plates Type B with inverse dome was in excess of 90% dose recovery.

The regular dome profile Type A generates a diverging aerosol plume. This diverging aerosol plume is generated by the convex shape dome of the aperture plates. The aerosol was seen to diverge the further it travelled away from the aperture plates.

In complete contrast, the aperture plates Type B have an inverse dome concave shape and the aerosol converged to generate a jet type stream of liquid. This jet type steam of liquid was not seen to diverge again for distances <0.5 meters. The narrow jet type steam of liquid appeared to have a span of less than 6 mm in diameters and this was confirmed by the high dose recovery.

Another important characteristic of the aperture plates was the flow rate. A minimum flowrate of 360 mL/hr was required to achieve the 10 µl in less than 0.1s.

The 15 µm conventional Type A aperture plates had a flowrate of -700 mL/hour, and the Type B aperture plates 15 µm INV2-had a flowrate -730 mL/hour.

The 20 µm conventional Type A aperture plates had a flowrate of -1340 mL/hour, and the Type B aperture plates 20 µm INV2-S1 and 20 µm INV2-S2 had flowrates -1550 mL/hour and 1500 mL/hour respectively.

### Example 2

The inversely domed aperture plates Type B 10, 15 & 20 µm were evaluated at a height of 3 and 4 cm from a target aperture and the aperture size was reduced from 8 to 6 mm. The results show that the height and the aperture hole size did not affect the dose delivery. The dose delivery was recovered across all parameters and ranged from 87.5±2.5%, 81±3% & 81±3% respectively.

In all cases of Type B aperture plates, the required dose delivery was achieved. All the aperture plates were nearly identical in dose delivery however the aperture plates are distinguished based on their flowrates. The flowrates increased proportionally to hole size. The 10, 15 & 20 µm Type B aperture plates had flowrates of 388, 700 and 1552 mL/hr respectively. The initial requirement for dose duration was ≤ 1 sec. preferably ≤ 0.5 sec, however this was reduced to 0.1 seconds to deliver the 10 µL dose (360 mL/hr) in an attempt to overcome the blink response. Again all the type B aperture plates met the requirement of 0.1 second. In this case of the 20 µm aperture plates the visual appearance of the aerosol jet was a more defined jet when compared to the 10 and 15 µm devices.

### Example 3

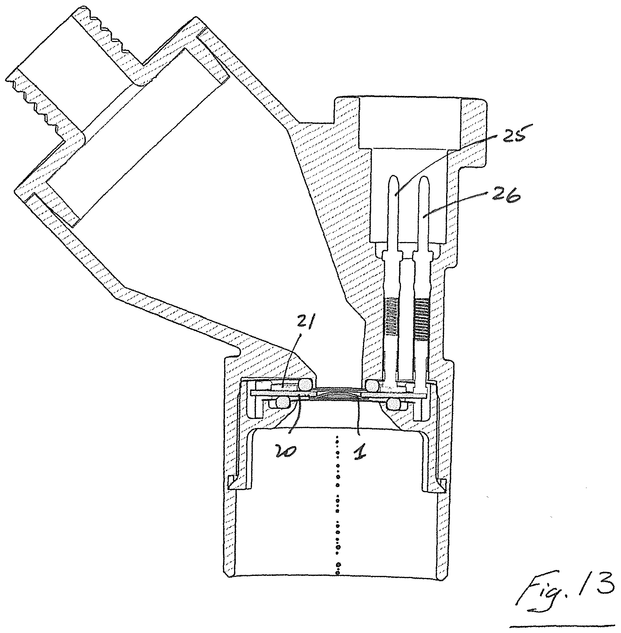

The flowrate of the 20 µm device was seen to be -1550 mL/hr, but when this was tested on a person's skin it was found to exert a strong force at the point of impact. In attempts to reduce the force of the jet, several reductions were made to the controller power output however it was seen, as power level decreased below 7 volts, the precision of the jet was affected. As a result of this finding the power output was set at 7 volts. The effect of reducing the power output resulted in a slower flow rate however it was still >>360 mL/hr. A novel approach of a pulsed algorithm was created to reduce the flowrate of the 20 µm device to -360 mL/hr. The algorithm was an on-off-on-off-etc. where the duration of the on/off cycle was <20 microseconds. This resulted in the reduction of the flowrate to the required 360 mL/hr. To the naked eye it still appeared as a one continuous jet, however utilising high speed camera analysis it was possible to see that the jet stream was made up of individual droplets caused by the on/off cycle as illustrated in Fig. 13. The force exerted by the jet was less than the same force sensation when applied to a person's skin.

The aerosol generators of the invention may be used in a wide range of applications including delivery to the eye and precision delivery devices more generally.

Advantages of the invention include:
- Precision dosing and targeting
- Control of mist, stream or Jet
- Programmable
- Low power consumption
- Fast flowrates
- Narrow aerosol plume

The invention overcomes at least some of the following problems with prior art device:
Aerosol droplet characteristics (too light and reflects off eye);
Dose Accuracy and precision (plume spreads too wide);
Speed of delivery (slow device flow rate, extends delivery).

Any suitable fluid may be aerosolised using the aperture plate of the invention. The fluid in one case is water. The fluid may be a solution which may contain an agent such as a diagnostic or therapeutic agent. For ophthalmic applications, the fluid may be any type of fluid that is pharmacologically compatible with the eye. In some cases the agent is selected from one or more of diagnostic agents, antibiotics, corticosteroids, antibiotic/corticosteroid combinations, lubricants, tear substitutes, tear production enhancement agents, decongestants, antihistamines, decongestant/antihistamine combination agents, antibacterial agents, antiviral agents, antimicrobial agents, steroidal anti-inflammatory agents, antibiotic/steroidal anti-inflammatory combination agents, nonsteroidal anti-inflammatory agents, topical anesthetic agents, topical anesthetic/fluorescein combination agents, hypertonic saline solution, mydriatic/cycloplegics, miotics, ocular hypotensive agents (anti-glaucoma agents) including: miotics, alpha-adrenergic agents, carbonic anhydrase inhibitors, beta-blocking agents, prostaglandin analogs, combination agents.

The invention is not limited to the embodiments hereinbefore described, which may be varied in construction and detail.

## Claims

1. An aerosol generator comprising:-
an aperture plate comprising:-
an inlet surface for receiving a liquid to be aerosolised,
an outlet surface, and
a plurality of apertures extending between an inlet opening at the inlet surface and an outlet opening at the outlet surface;
the aperture plate being convex in the direction of the inlet openings and concave in the direction of the outlet openings whereby aerosol generated on vibration of the aperture plate forms a converging plume;
a housing having an inlet for receiving a liquid to be aerosolised and an outlet through which the aerosol is delivered;
a support member for the aperture plate;
a vibration generating element for vibrating the aperture plate,
and an aperture plate drive circuit having a controller;
**characterised in that** the controller is configured to pulse the aperture plate in an on-off-on-off-manner.

2. An aerosol generator as claimed in claim 1 wherein the aperture plate is substantially dome shaped in geometry.

3. An aerosol generator as claimed in claim 1 or 2 wherein the apertures are tapered to narrow from the inlet opening at the inlet surface to an outlet opening adjacent to the outlet surface.

4. An aerosol generator as claimed in claim 3 comprising a flared portion adjacent to the exit opening.

5. An aerosol generator as claimed in any of claims 1 to 4 wherein the outlet openings are at least 10µm in diameter.

6. An aerosol generator as claimed in any of claims 1 to 5 wherein the outlet openings are from 10µm to 20µm, or from 15µm to 20µm in diameter.

7. An aerosol generator as claimed in any of claims 1 to 6 wherein the outlet openings are about 20µm in diameter.

8. An aerosol generator as claimed in any of claims 1 to 7 comprising a reservoir for liquid to be aerosolised.

9. An aerosol generator as claimed in claim 8 wherein the reservoir is defined by a portion of the housing.

10. An aerosol generator as claimed in any of claims 1 to 9 wherein the duration of one on-off cycle is less than 20 microseconds.

11. An aerosol generator as claimed in any of claims 1 to 10 wherein the support member comprises a central opening and wherein the aperture plate is mounted to the underside of the support member.

12. An ophthalmic fluid delivery device comprising an aerosol generator as claimed in any of claims 1 to 11.

## Patentansprüche

1. Aerosolerzeuger, der Folgendes umfasst:
eine Lochplatte, die Folgendes umfasst:
eine Einlassfläche zum Aufnehmen einer zu aerosolierenden Flüssigkeit,
eine Auslassfläche, und
eine Vielzahl von Löchern, die sich zwischen einer Einlassöffnung an der Einlassfläche und einer Auslassöffnung an der Auslassfläche erstrecken;
wobei die Lochplatte in der Richtung der Einlassöffnungen konvex ist und in der Richtung der Auslassöffnungen konkav ist, wodurch beim Vibrieren der Lochplatte erzeugtes Aerosol eine konvergierende Fahne bildet;
ein Gehäuse mit einem Einlass zum Aufnehmen einer zu aerosolierenden Flüssigkeit und einem Auslass, durch den das Aerosol freigesetzt wird;
ein Stützelement für die Lochplatte;
ein Vibrationserzeugungselement zum Vibrieren der Lochplatte, und
eine Lochplatten-Antriebsschaltung mit einer Steuerung;
**dadurch gekennzeichnet, dass** die Steuerung dazu konfiguriert ist, die Lochplatte auf Ein-Aus-Ein-Aus-Weise zu pulsieren.

2. Aerosolerzeuger nach Anspruch 1, wobei die Lochplatte eine im Wesentlichen kuppelförmige Geometrie aufweist.

3. Aerosolerzeuger nach Anspruch 1 oder 2, wobei die Löcher verjüngt sind, um von der Einlassöffnung an der Einlassfläche zu einer der Auslassfläche benachbarten Auslassöffnung enger zu werden.

4. Aerosolerzeuger nach Anspruch 3, umfassend einen der Austrittsöffnung benachbarten aufgeweiteten Abschnitt.

5. Aerosolerzeuger nach einem der Ansprüche 1 bis 4, wobei die Auslassöffnungen einen Durchmesser von mindestens 10 µm aufweisen.

6. Aerosolerzeuger nach einem der Ansprüche 1 bis 5, wobei die Auslassöffnungen einen Durchmesser von 10 µm bis 20 µm oder von 15 µm bis 20 µm aufweisen.

7. Aerosolerzeuger nach einem der Ansprüche 1 bis 6, wobei die Auslassöffnungen einen Durchmesser von ungefähr 20 µm aufweisen.

8. Aerosolerzeuger nach einem der Ansprüche 1 bis 7, umfassend einen Speicherbehälter für zu aerosolierende Flüssigkeit.

9. Aerosolerzeuger nach Anspruch 8, wobei der Speicherbehälter von einem Abschnitt des Gehäuses definiert wird.

10. Aerosolerzeuger nach einem der Ansprüche 1 bis 9, wobei die Dauer eines Ein-Aus-Zyklus weniger als 20 Mikrosekunden beträgt.

11. Aerosolerzeuger nach einem der Ansprüche 1 bis 10, wobei das Stützelement eine mittlere Öffnung umfasst und wobei die Lochplatte an der Unterseite des Stützelements befestigt ist.

12. Zuführvorrichtung für ophthalmisches Fluid, die einen Aerosolerzeuger nach einem der Ansprüche 1 bis 11 umfasst.

## Revendications

1. Générateur d'aérosol comprenant :
une plaque d'ouverture comprenant :
une surface d'entrée pour recevoir un liquide à mettre sous forme d'aérosol, une surface de sortie, et
une pluralité d'ouvertures s'étendant entre une ouverture d'entrée au niveau de la surface d'entrée et une ouverture de sortie au niveau de la surface de sortie ;
la plaque d'ouverture étant convexe dans la direction des ouvertures d'entrée et concave dans la direction des ouvertures de sortie, ce par quoi l'aérosol généré par vibration de la plaque d'ouverture forme un panache convergent ;
un boîtier ayant une entrée pour recevoir un liquide à mettre sous forme d'aérosol et une sortie par laquelle est délivré l'aérosol ;
un élément de support pour la plaque d'ouverture ;
un élément générateur de vibration pour faire vibrer la plaque d'ouverture,
et un circuit d'entraînement de plaque d'ouverture ayant un dispositif de commande ;
**caractérisé en ce que** le dispositif de commande est configuré pour donner des impulsions à la plaque d'ouverture de manière intermittente.

2. Générateur d'aérosol selon la revendication 1, dans lequel la plaque d'ouverture a une géométrie sensiblement en forme de dôme.

3. Générateur d'aérosol selon la revendication 1 ou 2, dans lequel les ouvertures sont effilées pour se rétrécir depuis l'ouverture d'entrée au niveau de la surface d'entrée jusqu'à une ouverture de sortie adjacente à la surface de sortie.

4. Générateur d'aérosol selon la revendication 3, comprenant une partie évasée adjacente à l'ouverture de sortie.

5. Générateur d'aérosol selon l'une quelconque des revendications 1 à 4, dans lequel les ouvertures de sortie ont un diamètre d'au moins 10 µm.

6. Générateur d'aérosol selon l'une quelconque des revendications 1 à 5, dans lequel les ouvertures de sortie ont un diamètre allant de 10 µm à 20 µm, ou allant de 15 µm à 20 µm.

7. Générateur d'aérosol selon l'une quelconque des revendications 1 à 6, dans lequel les ouvertures de sortie ont un diamètre d'environ 20 µm.

8. Générateur d'aérosol selon l'une quelconque des revendications 1 à 7, comprenant un réservoir pour un liquide à mettre sous forme d'aérosol.

9. Générateur d'aérosol selon la revendication 8, dans lequel le réservoir est défini par une partie du boîtier.

10. Générateur d'aérosol selon l'une quelconque des revendications 1 à 9, dans lequel la durée d'un cycle marche-arrêt est inférieure à 20 microsecondes.

11. Générateur d'aérosol selon l'une quelconque des revendications 1 à 10, dans lequel l'élément de support comprend une ouverture centrale et dans lequel la plaque d'ouverture est montée sur la face inférieure de l'élément de support.

12. Dispositif d'administration de fluide ophtalmique comprenant un générateur d'aérosol selon l'une quelconque des revendications 1 à 11.
